(19) Europäisches Patentamt
European Patent Office
Office européen des brevets

(11) **EP 3 150 998 B1**

(12) **EUROPEAN PATENT SPECIFICATION**

(45) Date of publication and mention
of the grant of the patent:
**08.01.2020 Bulletin 2020/02**

(21) Application number: **15800304.6**

(22) Date of filing: **26.05.2015**

(51) Int Cl.:
**G01N 33/38** *(2006.01)*      **G01N 27/02** *(2006.01)*

(86) International application number:
**PCT/ES2015/070410**

(87) International publication number:
**WO 2015/181422 (03.12.2015 Gazette 2015/48)**

(54) **EMBEDDED SENSOR FOR THE CONTINUOUS MEASUREMENT OF MECHANICAL RESISTANCE IN STRUCTURES MADE FROM CEMENTITIOUS MATERIAL, METHOD FOR PRODUCING THE SENSOR, AND SYSTEM AND METHOD FOR THE CONTINUOUS MEASUREMENT OF MECHANICAL RESISTANCE IN STRUCTURES MADE FROM CEMENTITIOUS MATERIALS**

EINGEBETTETER SENSOR ZUR KONTINUIERLICHEN MESSUNG DER MECHANISCHEN FESTIGKEIT IN STRUKTUREN AUS ZEMENTMATERIAL, VERFAHREN ZUR HERSTELLUNG DES SENSORS SOWIE SYSTEM UND VERFAHREN ZUR KONTINUIERLICHEN MESSUNG DER MECHANISCHEN FESTIGKEIT IN STRUKTUREN AUS ZEMENTMATERIALIEN

CAPTEUR INTÉGRÉ POUR LA MESURE EN CONTINU DE RÉSISTANCES MÉCANIQUES DANS DES STRUCTURES EN MATÉRIAU CIMENTAIRE, PROCÉDÉ DE FABRICATION DE CE DERNIER ET SYSTÈME ET PROCÉDÉ DE MESURE EN CONTINU DE RÉSISTANCES MÉCANIQUES DANS DES STRUCTURES EN MATÉRIAU CIMENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2014 ES 201430787**

(43) Date of publication of application:
**05.04.2017 Bulletin 2017/14**

(73) Proprietors:
• **Consejo Superior De Investigaciones Científicas (CSIC)**
  **28006 Madrid (ES)**
• **Pavol Jozef Safárik University in Kosice**
  **04180 Kosice (SK)**
• **The Technical University of Kosice**
  **04200 Kosice (SK)**
• **All World Certificacion, S.L.**
  **46110 Godella (Valencia) (ES)**

(72) Inventors:
• **OLIVERA CABO, Jesús**
  **28040 Madrid (ES)**
• **ANAYA VELAYOS, José Javier**
  **28040 Madrid (ES)**
• **GONZÁLEZ HERNÁNDEZ, Margarita**
  **28040 Madrid (ES)**
• **APARICIO SECANELLAS, Sofia**
  **28040 Madrid (ES)**
• **VARGA, Rastislav**
  **04180 Kosice (SK)**
• **ROVNAK, Marian**
  **04200 Kosice (SK)**
• **FUENTES RAMÍREZ, José Vicente**
  **46980 Paterna (Valencia) (ES)**

(74) Representative: **Pons Ariño, Angel**
  **Pons Patentes y Marcas Internacional, S.L.**
  **Glorieta Rubén Dario 4**
  **28010 Madrid (ES)**

(56) References cited:
**WO-A1-2007/116218      WO-A2-2010/151453**
**ES-A1- 2 333 575      US-A1- 2001 001 397**
**US-A1- 2007 144 618      US-A1- 2012 230 365**
**US-A1- 2012 230 365      US-B2- 7 771 545**

- V. ZHUKOVA ET AL: "Magnetic and Mechanical Properties of Magnetic Glass-Coated Microwires with Different Glass Coating", MATERIALS SCIENCE FORUM, vol. 480-481, 1 January 2005 (2005-01-01), pages 293-298, XP055239713, DOI: 10.4028/www.scientific.net/MSF.480-481.293
- V. ZHUKOVA ET AL.: 'Magnetic and mechanical properties of magnetic glass-coated microwires with different glass coating''.' MATERIALS SCIENCE FORUM, [Online] vol. 480-481, 2005, SWITZERLAND, pages 293 - 298, XP055239713 Retrieved from the Internet: <URL:http://www.scientific.net 2005 Trans Tech Publications>
- CHIRIAC H ET AL.: 'Amorphous glass-covered magnetic wires for sensing applications''.' SENSORS AND ACTUATORS A vol. 59, no. 1-3, 01 April 1997, LAUSANNE, CH, ISSN 0924-4247 pages 243 - 251, XP004117102
- MOHRI K ET AL. MAGNET DISPLACEMENT SENSOR USING AMORPHOUS WIRE ML ELEMENT FOR SENSING OF BIOMECHANICAL MOVEMENT. vol. 19930413, 13 April 1993, pages 19930413 - 19930416, XP055239717

## Description

## OBJECT OF THE INVENTION

The object of the present invention relates to a sensor for the continuous measurement of mechanical resistance in a structure made from cementitious material. Another object of the present invention relates to one measurement system using the aforementioned sensor, which consist of a continuous measurement system based on an electromagnetic induction effect, as well as to the measurement method of the system and a method for the production of said sensor.

The sensor is embedded directly, as a simple additional aggregate, in a structure made from cementitious material, preferably concrete, such as to allow the internal mechanical stress/deformation state of the structure to be measured continuously without affecting the structure or its stress state.

The sensor comprises a block of cementitious material in which a magnetic microwire is embedded, said magnetic microwire comprising a ferromagnetic core and an insulating glass sheath.

Once the sensor is embedded in the structure made from cementitious material, it allows the forces and deformations to which said structure made from cementitious material is subjected to be determined throughout the useful life thereof.

It is especially applicable in the construction industry.

## TECHNICAL PROBLEM TO BE SOLVED AND BACKGROUND OF THE INVENTION

The most commonly used method for the measurement of the resistance of concrete consists of laboratory tests that measure mechanical resistance on test pieces. The disadvantage lies in the fact that it is a destructive test. Among the most commonly used methods for the measurement of mechanical stresses or deformation are extensometer gauges. Said extensometer gauges are able to measure the mean longitudinal deformation and are mainly used attached to the outer surface, although nowadays there are some designs that allow them to be embedded in concrete structures. However, due to their particular design, they are very costly in comparison with a sensor simply produced from the same material that is to be embedded.

Fiber optic sensors and piezoelectric sensors are highly efficient and allow for the measurement of internal stresses and deformation. These sensors have a disadvantage in that they are very expensive, have a limited durability and require expensive measuring equipment, such as electronics and lasers, for their operation. Furthermore, they have a very narrow useful temperature range.

The development of microsensors allows functional magnetic materials with improved magnetic properties to be obtained at a low cost in order to be adapted to the requirements of applications in a wide range of industrial sectors.

However, amorphous magnetic microwires have drawn a great deal of attention due to their small dimensions, the possibility of adapting their magnetic properties in order to detect different magnitudes, and the simple processes of measuring and manufacturing associated with them.

One technological application of these magnetic microwires is based on the effect of magnetic bistability, and they may be used in numerous applications, making use mainly of the very sharp voltage spikes induced in small windings as a result of the process of propagation of reverse magnetization, as will be further explained in detail. These magnetic pulses have been used in several different applications, such as pulse generators, position and displacement sensors, magnetic field sensors, magnetoelastic sensors, revolution counters and goniometers, etc.

Therefore, the present invention provides a sensor that has the following advantages:

- it allows for the continuous measurement of mechanical resistance in any structure made from cementitious material,

- it allows for non-contact measurement of internal mechanical stresses in a structure made from cementitious material,

- it is embedded directly in the structure made from cementitious material to be studied without affecting the structure or the stress state,

- thermal treatments are applied which, on the one hand, allow for an improved stability of the sensor with regard to time, and on the other hand adapt the sensitivity of the sensor to the applied mechanical stresses in the range of applied mechanical stresses the sensor will be measuring.

Document US2012230365A1 discloses a temperature sensor comprising a microwire and a protecting tube, intended to be embedded in a cementitious structure for measuring temperature of the cementitious structure.

Document WO2007116218A1 relates to a method and apparatus for measuring physical quantities using amorphous ferromagnetic sensor elements, and wherein a change in switching field causes such materials exhibit the Barkhaussen effect seen as the material is subjected to stress.

The publication "Magnetic and Mechanical Properties of Magnetic Glass-Coated Microwires with Glass Coating" by V Zhukova (Materials Science Forum, vol. 480-481, pages 293-298), (retrievable at www.scientific.net/MSF.480-481.293, discloses glass coated microwires with two metallic nucleus compositions and three glass compositions.

Document US2007144618A1 discloses a soft magnetic alloy for microwire casting.

Document US7771545B2 relates to an amorphous metal alloy having high tensile strength and electrical resistivity.

## DESCRIPTION OF THE INVENTION

The present invention relates to an embedded sensor for the continuous measurement of mechanical resistance in structures made from cementitious material (preferably, the structure made from cementitious material is concrete), such that it comprises: at least one magnetic microwire, either amorphous or nanocrystalline, embedded in a block of cementitious material, where the magnetic microwire has a suitable composition so that it will not be affected by the alkaline environment of the block of cementitious material, and comprises a metal core. Preferably, the metal core is a ferromagnetic core.

According to one embodiment, the magnetic microwire comprises a ferromagnetic core and an insulating glass sheath that is resistant to the alkaline environment of the block of cementitious material, which, depending on the cementitious material from which said block is made up of, is selected from:

- a borosilicate glass sheath for high resistance to alkalis with at least 20-25% Zr, and
- a borosilicate glass sheath for a moderate resistance to alkalis with a low alkali content, such as "Pyrex", 74.5% $SiO_2$, 15% $B_2O_3$, 3% $Na_2O$, 2% $Al_2O_3$, 1.5% $K_2O$; or "Nonex", 73% $SiO_2$, 16.5% $B_2O_3$, 6% PbO, 3% $Na_2O$, 1.5% $K_2O$.

According to another embodiment, the magnetic microwire comprises a ferromagnetic core without the insulating glass sheath. In this case, the ferromagnetic core comprises a material selected from Chromium, Zirconium and a combination of the two in order to be resistant to corrosive and oxidizing agents of the block of cementitious material in which it will be embedded.

The present invention also relates to a system for the continuous measurement of mechanical resistance in structures made from cementitious material based on an electromagnetic induction effect, in that it comprises at least:

- one of the previously mentioned sensors where, in the case that the magnetic microwire comprises an insulating glass sheath, the ferromagnetic core comprises a chemical composition selected from CoFeSiB, FeSiB, FeNiSiB and FeCoSiB with a proportion of Si+B greater than 14% and lower than 35%, and of Co, Ni and Fe greater than 40%; where the magnetic microwire comprises a high and positive magnetostriction in a range of $3e^{-5}$ to $4e^{-5}$, a magnetic bistability, and where the sensor is embedded in the structure made from cementitious material;

- a drive coil;
- a sensor coil which is inside, and concentric to, the drive coil which generates a reversed field in its interior;
- an AC power supply connected to the drive coil; and
- a system for reading and displaying the reversed field generated in the sensor coil.

This system for continuous measurement based on an electromagnetic induction effect, in turn, is divided into another two measurement systems, depending on the depth at which the sensor is placed, consisting of, on the one hand a wireless measurement system in which the drive coil and the sensor coil are placed outside the structure made from cementitious material and, on the other hand, a measurement system with connecting wires in which the drive coil and the sensor coil are previously coiled around the sensor and embedded together with the sensor in the structure made from cementitious material.

The method for the continuous measurement of mechanical resistance in structures made from cementitious material based on the electromagnetic induction effect comprises:

- generating a voltage or alternating current by means of the AC power supply which generates a magnetic field in the drive coil where said magnetic field generates the propagation of a magnetic wall throughout the magnetic microwire of the sensor where said magnetic wall generates a narrow voltage pulse in a reversed field of the sensor coil,
- measuring the reversed magnetic field generated in the sensor coil by means of a system for reading and displaying the reversed magnetic field,
- establishing the mechanical resistance of the structure made from cementitious materials based on the measurement of the reversed magnetic field.

Lastly, the present invention also relates to a method for producing the sensor described, which comprises:

- producing the amorphous magnetic microwire by means of conventional ultra-rapid cooling and stretching techniques,
- protecting the magnetic microwire by means of a suitable composition so that it is resistant to the alkaline environment of the block of cementitious material,
- cutting the magnetic microwire into sections and subjecting it to a thermal treatment selected from an electric current and a conventional oven to stabilize the inner structure of said magnetic microwire, and improving the sensitivity of the magnetic microwire to applied mechanical stresses,
- applying a chemical treatment by means of acids to the cut ends of the magnetic microwire in order to obtain uniform and polished ends,

- embedding at least the magnetic microwire in a block of cementitious material; and
- curing the block of cementitious material, controlling the evolution of its mechanical properties by means of non-destructive assessment techniques.

## BRIEF DESCRIPTION OF THE DRAWINGS

To complete the description, and for the purpose of helping to make the characteristics of the invention more readily understandable, the specification is accompanied by a set of figures constituting an integral part of the same, which by way of illustration and not limitation represent the following:

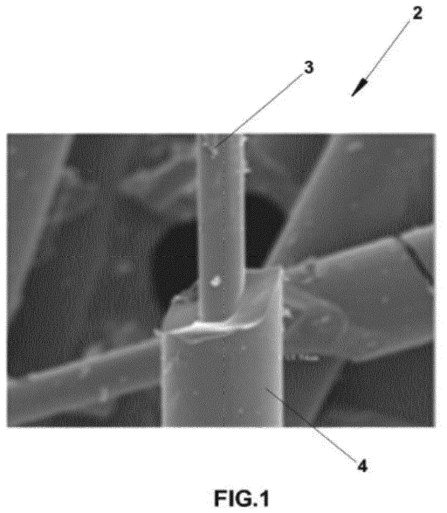

Figure 1 shows a micrograph of a magnetic microwire by means of electronic microscopy where the ferromagnetic core and the insulating glass sheath can be observed.

Figure 2 shows the structure of the magnetic domains of the microwire with a positive magnetostriction. It has a single magnetic monodomain in the direction of the axis of the magnetic microwire (axial) and radial magnetic domains near the surface.

Figure 2a shows the different magnetic states of the bistable magnetic hysteresis loop of a magnetic microwire with a positive magnetostriction. The figure to the left shows arrows that indicate the direction of the magnetization within each magnetic domain during the different states of the magnetization process corresponding to the stages of the magnetic hysteresis loop. H* represents the minimum magnetic field reversal necessary to be able to reverse the magnetization.

Figure 3 shows the signal induced in the sensor ($V_2$) together with the excitation voltage ($V_1$) as a function of time in arbitrary units (a.u.). The induced signal is due to the propagation of a magnetic domain wall throughout the magnetic microwire. The magnetic field reversal H* can be easily estimated based on the position of a sharp voltage spike (maximum).

Figure 4a shows a distribution of values of field H* of a composition of $Fe_{72.5}Si_{12.5}B_{15}$ before subjecting the magnetic microwire to thermal and chemical treatment. N is the number of events and H (Oe) the values of the magnetic field reversal. The mean value of the fluctuations is H*=0.83 A/m, the width of the curve is 0.25 Oe.

Figure 4b shows the previous figure after subjecting the magnetic microwire to the thermal and chemical treatment. It can be observed how the pulse-induced voltage is now more defined and stable, since the error in the measurement of a predetermined value of field H* has been reduced as much as possible.

Figure 5a shows an image of the surface imperfections at one end of the magnetic microwire (in this case the magnetic microwire does not have the insulating glass sheath) as a result of an imprecise cut; said image was taken with an optical microscope.

Figure 5b shows an image of one end of the magnetic microwire (in this case the magnetic microwire does have the insulating glass sheath); the image was taken with a scanning electron microscope (SEM).

Figure 6a shows an embodiment of a system based on a wireless electromagnetic induction effect. In this case, the drive coil and the sensor coil are outside the structure made from cementitious material and at a certain distance from the sensor embedded in the structure made from cementitious material.

Figure 6b shows an image of the magnetic microwire embedded in the block of cementitious material according to the measurement system based on the wireless electromagnetic induction effect.

Figure 6c shows an image taken with an optical microscope of the sensor (block of cementitious material together with the magnetic microwire) embedded in a structure made from cementitious material according to the measurement system based on the wireless electromagnetic induction effect.

Figure 6d shows an image taken with a scanning electron microscope (SEM) of the sensor in figure 6c.

Figure 7a shows an embodiment of a measurement system based on the electromagnetic induction effect with connecting wires.

Figure 7b shows the sensor (block of cementitious material together with the magnetic microwire) embedded in a structure made from cementitious material according to the measurement system based on the electromagnetic induction effect with connecting wires. The connecting wires that can be observed belong to the drive coil and sensor coil.

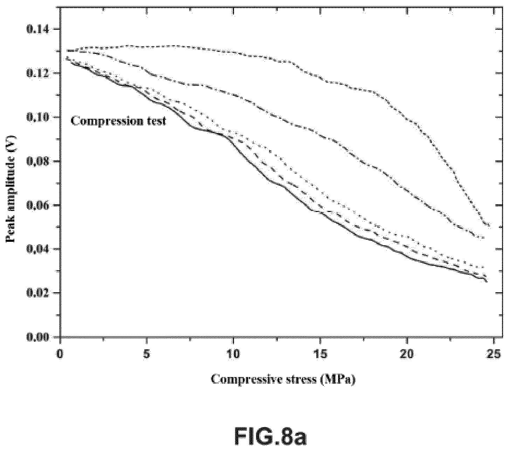

Figure 8a shows an electromotive pulse induced in the sensor coil as it is subjected to the block of cementitious material through compressive force. The dependence of the maximum position is observed for five specific cases, where the graphs show, from top to bottom, a charge for a single cycle (the line with the shortest and closest dashed lines), two cycles (the dashed lines with dots), four cycles (the short dashed line with longer spacing) six cycles (the line with the longest dashes) and eight cycles (continuous line).

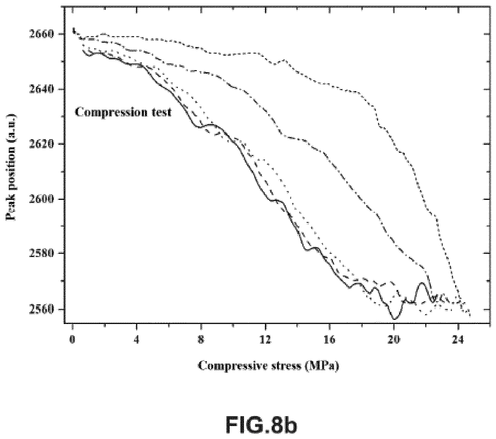

Figure 8b shows an electromotive pulse induced in the sensor coil as it is subjected to the block of cementitious material through compressive force. The dependence of their amplitude as a function of compressive force is observed for five specific measurements, where analogously to figure 8b, the graphs show, from top to bottom, a charge of a single cycle (the line with the shortest and closest dashed lines), two cycles (the dashed lines with dots), four cycles (the short dashed line with more spacing) six cycles (the line with the longest dashes) and eight cycles (continuous line).

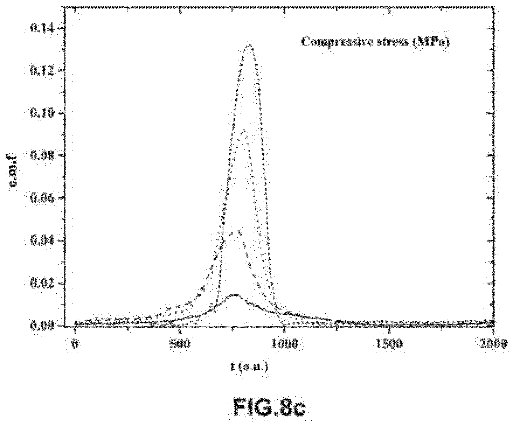

Figure 8c shows a schematic drawing of the change in position and amplitude of the induced pulse with regard to the compressive force for four cases.

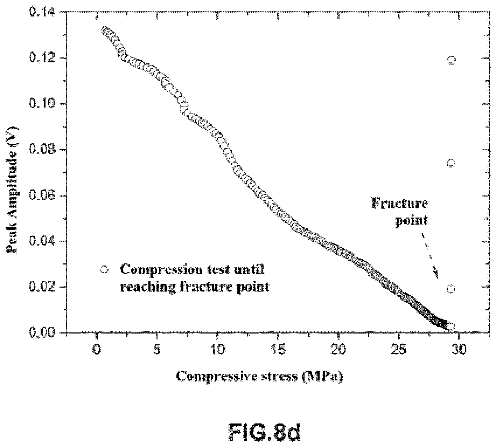

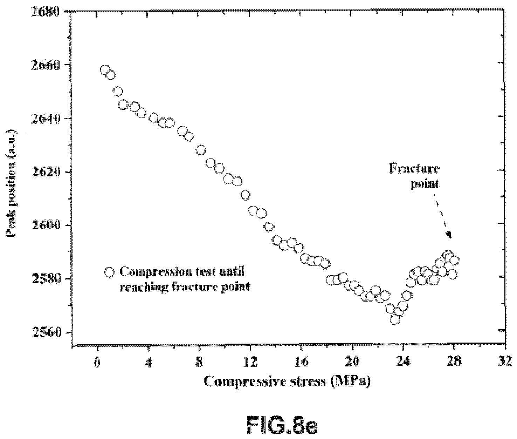

Figure 8d and 8e show compression tests like those of figures 8a and 8b, respectively, carried out until the fracture point was reached for the test piece.

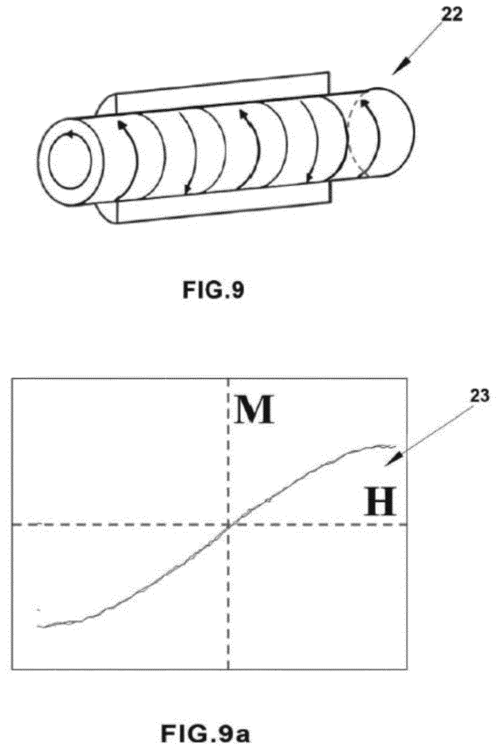

Figure 9 shows the structure of the magnetic domains of a microwire with a negative magnetostriction. It has mainly circular domains.

Figure 9a schematically shows the magnetic hysteresis loop which is characteristic of a magnetic microwire with a negative magnetostriction, flat and without hysteresis.

Below is a list of the different elements shown in the figures that are included in the invention:

1. Sensor.
2. Magnetic microwire.
3. Ferromagnetic core.
4. Insulating glass sheath.
5. Block of cementitious material.
6. Structure made from cementitious material.
7. Drive coil.
8. Sensor coil.
9. Damping coil.
10. Magnetic field reversal.
11. Connecting wires.
12. Function generator.
13. Digital oscilloscope.
14. Resistance.
15. Frequency histogram.
16. Excitation field.
17. Signal obtained from the magnetic field reversal.
18. Curves of magnetic inductance.
19. Electromotive pulse induced in the sensor coil.
20. Structure of the magnetic domains of a magnetic microwire with a positive magnetostriction.
21. Bistable magnetic hysteresis loop of a magnetic microwire with
a positive magnetostriction.
22. Structure of the magnetic domains of a magnetic microwire with
a negative magnetostriction.
23. Magnetic hysteresis loop of a magnetic microwire with
a negative magnetostriction.

## DETAILED DESCRIPTION

The sensor (1), object of the present invention, allows for the continuous measurement of mechanical resistance in any structure (6) made from cementitious material. Said sensor (1) is embedded directly, as an additional aggregate, in the structure (6) made from cementitious material to be studied (which will preferably be concrete), without affecting the structure (6) or its stress state, such as to allow for a continuous monitoring of its internal mechanical stress/deformation state.

Said sensor (1) of compressive forces comprises a block (5) of cementitious material in which the magnetic microwire (2) is embedded, which does not necessarily need to be made from the same cementitious material that makes up the structure (6) made from cementitious material to be studied, with the possibility of being made up of cement paste, mortar or concrete.

Said sensor (1) is able to continuously monitor the level of compressive force to which the structure (6) made from cementitious material to be studied will be subjected.

The magnetic microwire (2) may be either amorphous or nanocrystalline and has a suitable composition so as not to be affected by the alkaline environment of the block (5) of cementitious material. According to a preferred embodiment, the magnetic microwire (2) comprises a metal core (3) and an insulating glass sheath (4). Preferably, the metal core (3) is a ferromagnetic core (3), which henceforth shall be referred to as ferromagnetic core (3).

The insulating glass sheath (4) is chosen to be resistant to the alkaline environment of the block (5) of cementitious material, which, depending on the cementitious material of which said block (5) is made up, is selected from:

- a borosilicate glass sheath for high resistance to alkalis with at least 20-25% Zr, and
- a borosilicate glass sheath for a moderate resistance to alkalis with a low alkali content, such as the "Pyrex" type 74.5% $SiO_2$, 15% $B_2O_3$, 3% $Na_2O$, 2% $Al_2O_3$, 1.5% $K_2O$, or "Nonex" type, 73% $SiO_2$, 16.5% $B_2O_3$, 6% PbO, 3% $Na_2O$, 1.5% $K_2O$.

It is also possible to produce magnetic microwires (2) without the insulating glass sheath (4). In this case, the ferromagnetic core (3) comprises a material selected from Chromium, Zirconium and a combination of the two in order to be resistant to corrosive and oxidizing agents of the block (5) of cementitious material in which it will be embedded.

It is understood that corrosive agents refer to those that are caused by the very hydration of the concrete which contains water and which can oxidize the magnetic microwire (2), or those that can penetrate from outside, such as chlorides or sulphates.

The block (5) of cementitious material in which the magnetic microwire (2) is embedded preferably has a cylindrical form, since it facilitates a calibration of the sensor (1). However, it may have any other geometric form.

Once the block (5) made from cementitious material is manufactured and the magnetic microwire (2) is embedded, the sensor (1) is cured in conditions of water with calcium hydroxide for 28 days. During the curing process of the block (5) made from cementitious material in which the magnetic microwire (2) is embedded, the evolution of its mechanical properties has been controlled by means of measuring the ultrasonic velocity.

Due to the microscopic diameter of the magnetic microwire (2), 1 to 100 microns, the magnetic microwire (2) does not influence the elastic and mechanical properties of the block (5) made from cementitious material in which it is embedded.

It is important to mention that the range of forces for which the sensor (3) operates for a continuous monitoring of a compression-deformation curve is determined by controlling the following variables of the magnetic microwire (2):

i) Its sensitivity can be modified to a specific range of mechanical forces, for a given chemical composition, on the one hand modifying its structure and magnetostriction by means of specific thermal treatments, which can modify its magnetic-elastic response. They can be summarized by the following: thermal treatment with direct current or in a conventional thermal oven simultaneously with a magnetic field or applied mechanical stress, on the other hand, changing the diameter of the ferromagnetic core (3) and the thickness of the insulating glass sheath (4) of the magnetic microwire (2).

ii) Its mechanical resistance to the compression of the block (5) made from cementitious material can be modified, varying the manufacturing conditions of the block (5) made from cementitious material by means of the water/cementitious material ratio, the type of cementitious material, and aggregates, additives, as well as the curing conditions of said block (5) made from cementitious material.

iii) The dimensions of the block (5) made from cementitious material can be modified, since by modifying the cross section of the block (5) made from cementitious material, the mechanical stresses that affect the magnetic microwire (2) are also modified.

The manufacturing of these magnetic microwires (2) is done by means of the already known ultra-rapid cooling technique developed by Taylor-Ulitovski, in which, from one gram of an alloy, several kilometers of magnetic microwire (2) with an amorphous structure may be obtained. The amorphous structure is achieved thanks to the highly rapid cooling of the melted alloy (1250 °C) which is approximately $10^5$-$10^6$ °C/s.

The cylindrical form of the solidified magnetic microwire (2) is the result of the symmetry of the cooling process over water.

To optimize the response of the sensor (1), thermal treatments have been carried out after the magnetic microwire (2) has been cut, since if the magnetic microwire (2) is thermally treated and then later cut, the magnetic domain wall of the magnetic microwire (2) is destabilized. Said thermal treatments are applied to the entire magnetic microwire (2) with electric current or in a conventional oven simultaneously with a magnetic field or applied mechanical stress.

Additionally, chemical treatments have been carried out by means of acids such as HF, HCl and $H_2SO_4$ to the cut ends of the magnetic microwire (2) after the thermal treatments, to go from an end with imperfections where the roughness is a result of the traces of the tool used for cutting the magnetic microwire (2), to a more polished and uniform end.

There are mainly two systems for the continuous measurement of mechanical resistance in structures (6) made from cementitious materials:

- a first measurement system based on an electromagnetic induction effect which, in turn, is divided into another two measurement systems depending on the depth at which the sensor (1) is placed and, on the other hand, a measurement system with connecting wires (11); and
- a second measurement system based on the magnetic inductance (MI) effect.

Only the first measurement system is explained below:

A measurement system based on the electromagnetic induction effect:

The measurement system based on the electromagnetic induction effect comprises a power supply that generates a voltage or alternating current in a drive coil (7) which generates a magnetic field that causes the propagation of a magnetic domain wall throughout the magnetic microwire (2) from its end, inducing a narrow voltage pulse as it passes through the sensor coil (8), where the position of the spike of the narrow voltage pulse depends on the mechanical stresses applied to the magnetic microwire (2).

In this case, the magnetic/elastic behaviour of the magnetic microwires (2) can be modified, choosing a suitable composition, with a high magnetostriction so that it will be very sensitive to applied mechanical forces, of both stress and compression.

For this measurement system, the magnetic microwire (2) has specific properties:

- Compositions of CoFeSiB, FeSiB, FeNiSiB, FeCoSiB with a proportion of Si+B greater than 14% and lower than 35%, and of Co, Ni and Fe greater than 40%. Other elements may be added, such as: Mo, Zr, Ge, Cr, Mn, V, Ti, C, Cu, Nb or other metals or metalloids with contents lower than 7%,
- a chemical composition in order to have a high and positive magnetostriction in the range of $3e^{-5}$ to $4e^{-5}$ (30-40 ppm, parts per million),
- magnetic bistability
- geometric relation of the ferromagnetic core (3)/insulating glass sheath (4) so that it is not sensitive to the contraction forces of the cementitious material but is sensitive to the range of forces for which the sensor (1) operates,

- the magnetic microwire (2), before being embedded in the block of (5) cementitious material, has been subjected to:

 ○ a chemical treatment applied to the ends of the magnetic microwire (2) by means of acids such as HF, HCl and $H_2SO_4$ to go from an end with imperfections where the roughness is a result of the traces of the tool used for cutting the magnetic microwire (2) to a more polished and uniform end,

 ○ a thermal treatment for stabilization of the magnetic wall in a temperature range between 200 and 400 °C for approximately one hour and the corresponding chemical treatment to the ends. This treatment provides the following advantages:

  ▪ it stabilizes the magnetic wall of the microwire, reducing the fluctuations of the reversed field, which improves the precision of the sensor (1).

 ○ a thermal treatment with electric current or in a conventional oven in a temperature range between 200-600 °C for time

 periods that range from minutes to one hour, depending on each type of sample and treatment, simultaneously with the force of stress or magnetic field. This thermal treatment provides the following advantages:

  ▪ it stabilizes the inner structure of the magnetic microwire (2) over time in order to improve the stability of the sensor (1),

  ▪ it relaxes the internal stresses generated during manufacturing (at the same time, by means of the application of the magnetic field or mechanical stress, new stresses are introduced) which allow for an improvement in its axial magnetization in the direction of the axis of the magnetic microwire (2) and, therefore, establish the operational range of the same so that it has a sensitivity to the mechanical stresses applied in the axial direction of the magnetic microwire (2) for that range,

  ▪ for specific values of current and temperature it can even change its amorphous structure to nanocrystalline, also changing its magnetic-elastic behavior, translating to a greater or lesser dependence on the magnetic fields or applied mechanical stresses.

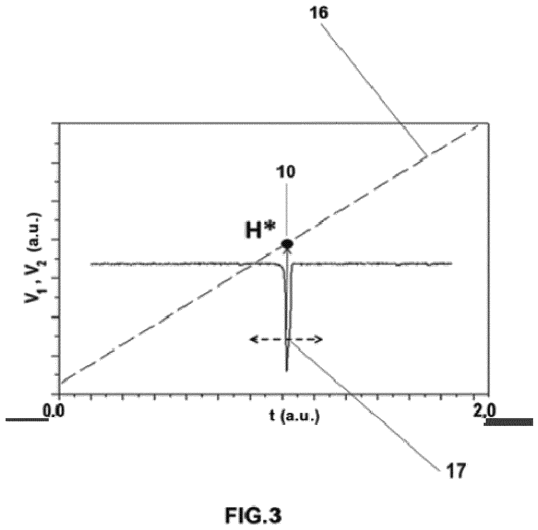

The advantage of the magnetization process in magnetic microwires (2) of a single Barkhausen jump, also known as bistable magnetic microwires (2), is that a simple induction method for a magnetic field reversal H* (10) can be used. When the external magnetic field exceeds the reversed magnetic field H* (10) caused by a drive coil (7) powered by a signal, a wall of magnetic domains propagates from its end throughout the magnetic microwire (2), achieving an electromotive force (e.m.f) induced in a sensor coil (8). Said wall of propagating magnetic domains can be seen in an oscilloscope as a sharp voltage spike, as can be observed in figure 3. The reversed magnetic field H* (10) can be easily estimated from the position of that maximum.

The sensor (1) can be influenced by local magnetic fields, or by metallic materials in its surroundings, such as in the reinforced concrete, and given that the objective of the sensor (1) is to be embedded in any structure (6) made from cementitious material to continuously monitor stresses, it is necessary to eliminate the parasitic magnetic fields caused by the Earth's magnetic field, as well as the metallic materials in its surroundings. Therefore, the measured reversed magnetic field H* (10) is measured in both directions of the applied magnetic field excitation. Therefore, a reversed magnetic field $H^*_+$ (10) will be measured when the magnetic field excitation increases and $H^*_-$ when it decreases. Such that the reversed magnetic field H* (10) which will be used to measure the applied forces (independent from the local field) is proportional to the difference of both components:

$$H^* = \frac{H^*_+ - H^*_-}{2}$$

while the local or parasitic magnetic field will be represented by:

$$H^*_{local} = \frac{H^*_+ + H^*_-}{2}$$

Additionally, the sensor (1), once embedded in a structure of cementitious material, preferably concrete, can be used to measure the temperature in extreme cases when the sensor (1) is subjected to drastic temperature changes. Lastly, if it is desirably to avoid a possible dependency on the temperature, it is only necessary to modify the measuring conditions of the sensor (1).

It has been demonstrated that at low frequencies for the excitation signal, the sensor (1) has a small sensitivity to mechanical stress and this can be used, for example, as a warning of abrupt temperature changes which can be dangerous to the integrity of the structure made from cementitious material (6). If the frequency of the magnetic field increases, an increase in the sensitivity of the sensor (1) to the mechanical stress is produced.

The alloy of the ferromagnetic core (3) of the magnetic microwire (2) is carefully chosen so that it has a high magnetostriction constant for the measurement of the reversed magnetic field H* (10). Magnetostriction is the property of the ferromagnetic materials that deform in the presence of magnetic fields. Here the inverse effect is used, known as the Villari effect, which causes variations in magnetic properties (in our case, the effects of the reversed field) dependent on the mechanical force applied to them.

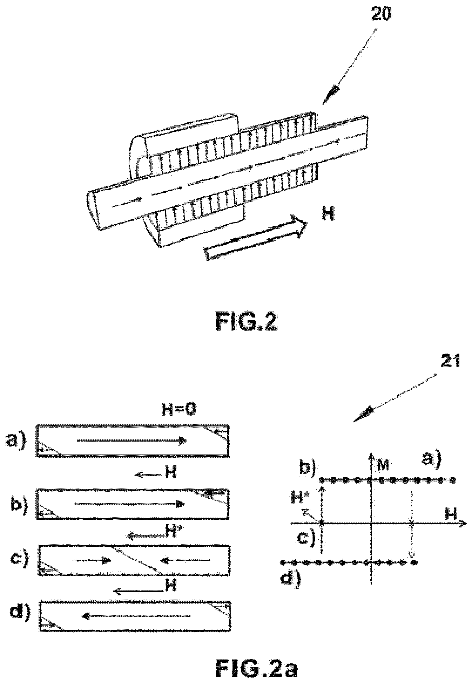

For the measurement of the reversed magnetic field (10) H*, the magnetic microwires (2) have a particular magnetic structure, displaying characteristics of magnetic bistability. And a magnetization reversal is produced that goes from one stable energy state to another by means of a Barkhausen jump. Figure 2 shows a schematic representation of the magnetic monodomain structure. In figure 2a, the arrows indicate the direction of the magnetization within each domain during the different states of the magnetization process which correspond to the stages of a completely rectangular magnetic hysteresis loop characteristic of a bistable magnetic microwire (2) with a positive magnetostriction. The change in magnetization when a magnetic field H is gradually applied is greater in the opposite direction than how it is magnetized in state a) to field H*, where only a single Barkhausen jump takes place from the magnetic wall formed at the end of the magnetic microwire (2) in state c) until finally achieving state d). Field H* represents the minimum reverse magnetic field necessary to be able to invert the magnetization.

In the remanence (a), when a magnetic field is applied in the opposite direction to how the magnetized core is axially in (a) the structures of existing closure domains, they extend towards the center of the sample. (b) In the reverse field, the wall of one of the two closure domains disengages in an irreversible way and moves towards the opposite end of the sample, giving rise to a change in magnetization (c). Therefore, said Barkhausen jump is the result of the disengaging and propagation of a single wall of magnetic domains throughout the magnetic microwire (2). This propagation is defined by a narrow magnetic pulse of a specific duration (figure 3).

However, the value of the reversed magnetic field h* in which the magnetization jump is produced has a small fluctuation upon being repeatedly measured in the same experimental conditions. To achieve a good sensitivity, it is necessary to have a large amplitude and a short duration, and the distribution should tend to be as narrow as possible.

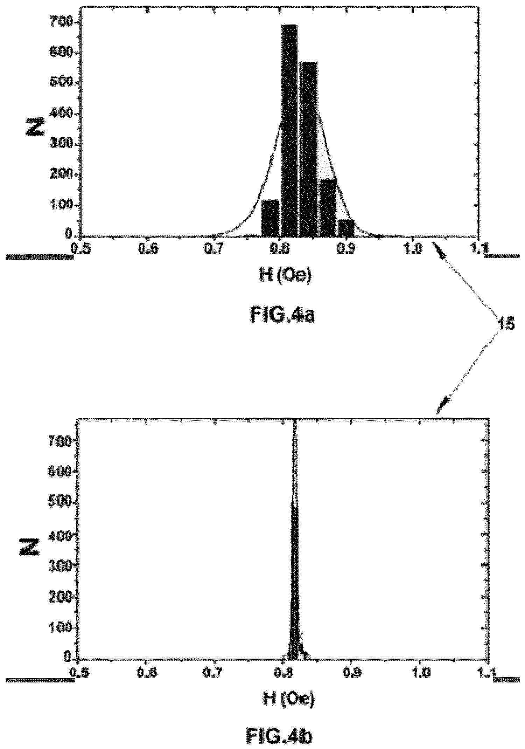

One of the novelties of this measurement system based on the electromagnetic induction effect is that improvements have been made in the stabilization of the magnetic domain wall of the magnetic microwire (2), thereby achieving an optimization in the response of the sensor (1) with greater precision in its measurements. In this way, it has been possible to go from the distribution of measurements of the reverse magnetic field H* (10) in figure 4a to the more stable values in figure 4b. This way, a more stable spike is achieved, which practically does not change its position over time.

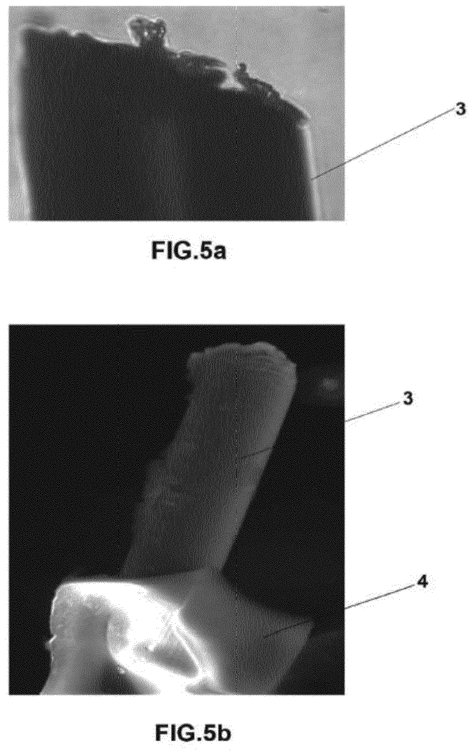

Figure 5a shows an image of the surface imperfections due to an imprecise cut at the end of the magnetic microwire (2) without an insulating glass sheath (4), taken with an optical microscope, while figure 5b shows an image of the end of the magnetic microwire (2) with the insulating glass sheath (4) taken by means of a scanning electronic microscope (SEM).

The methodology of acquiring and analyzing data is done in accordance with conventional methods where a data acquisition program calculates the maximum value of an induced voltage (in mV) and stores the time in which this maximum has been produced. This routine of recording the maximum position is repeated for every reversed magnetic field H* (10) value one wants to measure.

Tests have been done with untreated samples, meaning samples to which the aforementioned treatments have not been applied (statistics have been done for two hundred data on reversed magnetic fields (10)) and they have been compared to samples to which the aforementioned treatments were applied, and it has been clearly observed that there is a substantial reduction in the fluctuations and a value of the reversed magnetic field that is practically stable around the mean value of the approximation curve.

As was mentioned previously, depending on the accessibility to the area of the structure (6) made from cementitious material where the sensor (1) is placed, two different measurement systems will have to be used.

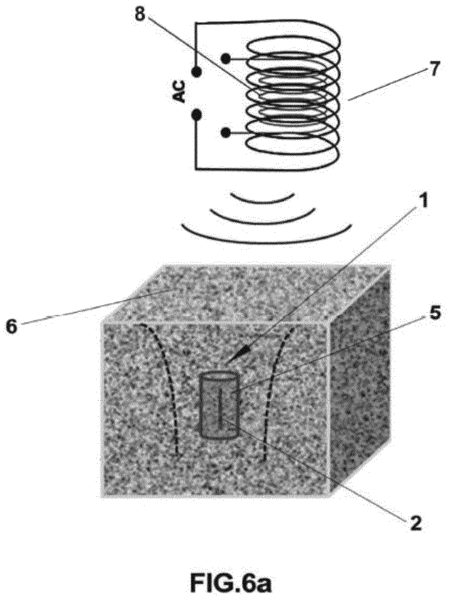

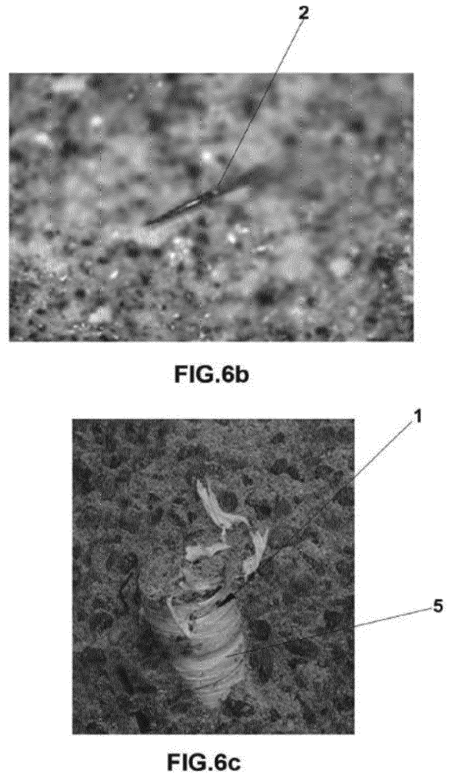

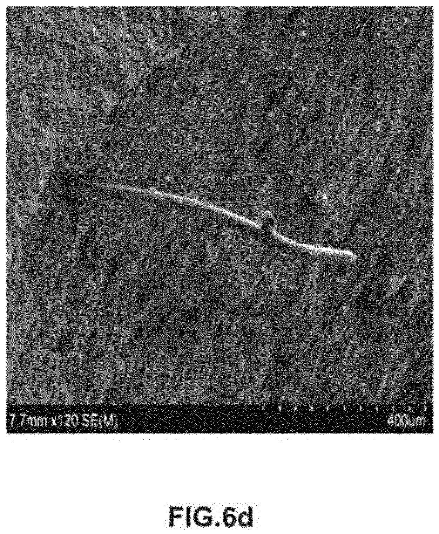

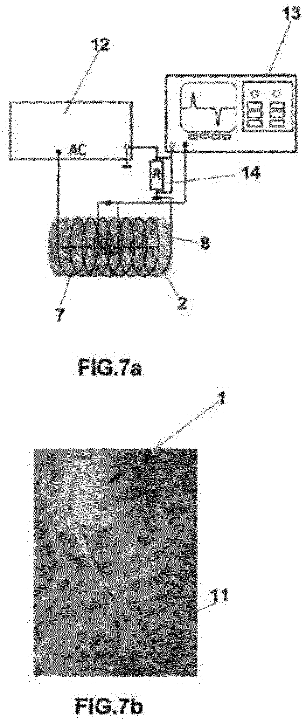

- If the area is accessible and the embedded sensor (1) is in the proximity of the surface of the structure (6) made from cementitious material, the measurement system based on the wireless electromagnetic induction effect is used, as can be observed in figure 6a, where a drive coil (7) and a sensor coil (8) are placed outside the structure (6) made from cementitious material, whereas the sensor (1) is embedded inside said structure (6) made from cementitious material. The embedded sensor (1) has been previously calibrated in a laboratory through experiments of force vs. the measurement of magnetic parameters. Once calibrated, the sensor (1) can be added as an additional aggregate in the manufacturing of the structure (6) made from cementitious material in which the state of forces is to be measured. The sensor (1) must be oriented in the direction in which the stresses are to be measured.
  The drive coil (7) is powered by a signal, preferably in a sinusoidal or triangular form, with the aim of applying a linear or gradual external magnetic field that increases over time.
- If the area is not easily accessible, the measurement system based on the electromagnetic induction effect with connecting wires (11) is used, as can be observed in figure 7a, where a drive coil (7) and a sensor coil (8) are embedded and coiled around the block (5) of cementitious material in which the magnetic microwire (2) is embedded. Connecting cables (11) come out of the two coils, the drive coil (7) and the sensor coil (8), as shown in figure 7b.

The connecting wires (11) are fine co-axial wires that are coated, preferably, with polytetrafluoroethylene (PTFE) to prevent them from being damaged when embedded in the block (5) made from cementitious material.

The above-explained measurement system is easy to electronically instal, which would allow for the use of systems based on wireless networks for sensors or RFID technology to monitor the properties of the structure (6) made from cementitious material being studied.

The present invention should not be taken to be limited to the embodiment herein described. Other arrangements may be carried out by those skilled in the art based on the present description. As such, the scope of the invention is defined by the following claims.

**Claims**

1. An embedded sensor (1) for the continuous measurement of mechanical resistance in structures (6) made from cementitious material **characterized in that** it comprises:

   - a block (5) of cementitious material, configured to be embedded in structures (6) made from cementitious material, and
   - at least one amorphous magnetic microwire (2), embedded in the block (5) of cementitious material, where the magnetic microwire (2) is resistant to the alkaline environment of the block (5) of cementitious material and comprises a metal core (3).

2. An embedded sensor (1) for the continuous measurement of mechanical resistance in structures (6) made from cementitious material **characterized in that** it comprises:

   - a block (5) of cementitious material, configured to be embedded in structures (6) made from cementitious material, and
   - at least one nanocrystalline magnetic microwire (2), embedded in the block (5) of cementitious material, where the magnetic microwire (2) is resistant to the alkaline environment of the block (5) of cementitious material and comprises a metal core (3).

3. The embedded sensor (1) for the continuous measurement of mechanical resistance in structures (6) made from cementitious material according to any one of the preceding claims, **characterized in that** the metal core (3) is ferromagnetic.

4. The embedded sensor (1) for the continuous measurement of mechanical resistance in structures (6) made from cementitious material according to claim 3, **characterized in that** the ferromagnetic core (3) comprises a material selected from Chromium, Zirconium and a combination of the two in order to be resistant to corrosive and oxidizing agents of the block (5) of cementitious material.

5. The embedded sensor (1) for the continuous measurement of mechanical resistance in structures (6) made from cementitious material according to claim 3, **characterized in that** the microwire (2) further comprises an insulating glass sheath (4) in order to be resistant to the environment of the block (5) of cementitious material.

6. The embedded sensor (1) for the continuous measurement of mechanical resistance in structures (6) made from cementitious material according to claim 5, **characterized in that** the insulating glass sheath (4) is a borosilicate glass sheath.

7. The embedded sensor (1) for the continuous measurement of mechanical resistance in structures (6) made from cementitious material according to claim 6, **characterized in that** the borosilicate glass sheath comprises a composition selected from

   - 74.5% $SiO_2$, 15% $B_2O_3$, 3% $Na_2O$, 2% $Al_2O_3$, 1.5% $K_2O$; and
   - 73% $SiO_2$, 16.5% $B_2O_3$, 6% $PbO$, 3% $Na_2O$, 1.5% $K_2O$.

8. The embedded sensor (1) for the continuous measurement of mechanical resistance in structures (6) made from cementitious material according to claim 6, **characterized in that** the borosilicate glass sheath comprises a composition of at least 20-25% Zr.

9. The embedded sensor (1) for the continuous measurement of mechanical resistance in structures (6) made from cementitious material according to claim 5, **characterized in that** the ferromagnetic core (3) comprises a chemical composition selected from CoFeSiB, FeSiB, FeNiSiB, and FeCoSiB with a proportion of Si+B greater than 14% and lower than 35%, and of Co, Ni and Fe greater than 40%.

10. The embedded sensor (1) for the continuous measurement of mechanical resistance in structures (6) made from cementitious material according to claim 5, **characterized in that** the ferromagnetic core (3) comprises a chemical composition selected from CoSiB and CoFeSiB with a proportion of Si+B greater than 15% and lower than 35%, of Co greater than 40% and Fe lower than 6%.

11. A system for the continuous measurement of mechanical resistance in structures (6) made from cementitious material based on an electromagnetic induction effect, **characterized in that** it comprises:

- the sensor (1) as described in any one of claims 1 to 10, where the magnetic microwire (2) comprises a high and positive magnetostriction in a range of $3e^{-5}$ to $4e^{-5}$, and a magnetic bistability, and wherein the sensor (1) is configured to be embedded in structures (6) made from cementitious material;
- a drive coil (7);
- a sensor coil (8) located inside, and concentrically to, the drive coil (7) which generates a reversed field in its interior;
- an AC power supply connected to the drive coil (7); and
- a system for reading and displaying the reversed field generated in the sensor coil (8).

12. A method for the continuous measurement of mechanical resistance in structures (6) made from cementitious material based on the electromagnetic induction effect which makes use of the measurement system defined in claim 11, **characterized in that** it comprises:

- generating an alternating voltage or an alternating current by means of the AC power supply which generates a magnetic field in the drive coil (7) where said magnetic field generates the propagation of a magnetic wall throughout the magnetic microwire (2) of the sensor (1), wherein said magnetic wall generates a narrow voltage pulse in a reversed magnetic field of the sensor coil (8),
- measuring the reversed magnetic field (10) generated in the sensor coil (8) through a system for reading and displaying the reversed magnetic field (10),
- establishing the mechanical resistance of the structure (6) made from cementitious material based on the measurement of the reversed magnetic field (10).

13. The method for the continuous measurement of mechanical resistance in structures (6) made from cementitious material based on the electromagnetic induction effect according to claim 12, **characterized in that** the drive coil (7) and the sensor coil (8) stay outside the structure (6) made from cementitious material.

14. The method for the continuous measurement of mechanical resistance in structures (6) made from cementitious material based on the electromagnetic induction effect according to claim 12, **characterized**

in that the drive coil (7) and the sensor coil (8) are previously coiled around the sensor (1) and embedded, together with the sensor (1), in the structure (6) made from cementitious material.

15. A method for producing the embedded sensor (1) defined in any one of claims 1 and 3 to 10, **characterized in that** it comprises:

- producing the amorphous magnetic microwire (2) by means of conventional ultra-rapid cooling and stretching techniques,
- protecting the magnetic microwire (2) by means of a suitable composition so that it is resistant to the alkaline environment of the block (5) of cementitious material,
- cutting the magnetic microwire (2) into sections and subjecting it to a thermal treatment selected from between using an electric current and using a conventional oven to stabilize the inner structure of said magnetic microwire (2), and improving the sensitivity of the magnetic microwire (2) to applied mechanical stresses,
- applying a chemical treatment by means of acids to the cut ends of the magnetic microwire (2) in order to obtain uniform and polished ends,
- embedding at least the magnetic microwire (2) in a block (5) of cementitious material; and
- curing the block (5) of cementitious material, controlling the evolution of its mechanical properties by means of non-destructive assessment techniques.

**Patentansprüche**

1. Eingebetteter Sensor (1) zur kontinuierlichen Messung einer mechanischen Festigkeit von Strukturen (6), die aus Zementmaterial gefertigt sind, **dadurch gekennzeichnet, dass** er umfasst:

- einen Block (5) aus Zementmaterial, der konfiguriert ist, in Strukturen (6) aus Zementmaterial eingebettet zu werden, und
- mindestens einen amorphen magnetischen Mikrodraht (2), der in den Block (5) aus Zementmaterial eingebettet ist, wobei der magnetische Mikrodraht (2) gegenüber der alkalischen Umgebung des Blocks (5) aus Zementmaterial beständig ist und einen Metallkern (3) umfasst.

2. Eingebetteter Sensor (1) zur kontinuierlichen Messung einer mechanischen Festigkeit von Strukturen (6), die aus Zementmaterial gefertigt sind, **dadurch gekennzeichnet, dass** er umfasst:

- einen Block (5) aus Zementmaterial, der konfiguriert ist, in Strukturen (6) aus Zementmaterial eingebettet zu werden, und

- mindestens einen nanokristallinen magnetischen Mikrodraht (2), der in den Block (5) aus Zementmaterial eingebettet ist, wobei der magnetische Mikrodraht (2) gegenüber der alkalischen Umgebung des Blocks (5) aus Zementmaterial beständig ist und einen Metallkern (3) umfasst.

3. Eingebetteter Sensor (1) zur kontinuierlichen Messung einer mechanischen Festigkeit von Strukturen (6), die aus Zementmaterial gefertigt sind, nach einem der vorstehenden Ansprüche, **dadurch gekennzeichnet, dass** der Metallkern (3) ferromagnetisch ist.

4. Eingebetteter Sensor (1) zur kontinuierlichen Messung einer mechanischen Festigkeit von Strukturen (6), die aus Zementmaterial gefertigt sind, nach Anspruch 3, **dadurch gekennzeichnet, dass** der ferromagnetische Kern (3) ein Material umfasst, das ausgewählt ist aus Chrom, Zirkonium und einer Kombination der beiden, um gegenüber Korrosions- und Oxidationsmitteln des Blocks (5) aus Zementmaterial beständig zu sein.

5. Eingebetteter Sensor (1) zur kontinuierlichen Messung einer mechanischen Festigkeit von Strukturen (6), die aus Zementmaterial gefertigt sind, nach Anspruch 3, **dadurch gekennzeichnet, dass** der Mikrodraht (2) ferner eine Isolierglasummantelung (4) umfasst, um gegenüber der Umgebung des Blocks (5) aus Zementmaterial beständig zu sein.

6. Eingebetteter Sensor (1) zur kontinuierlichen Messung einer mechanischen Festigkeit von Strukturen (6), die aus Zementmaterial gefertigt sind, nach Anspruch 5, **dadurch gekennzeichnet, dass** die Isolierglasummantelung (4) eine Borsilikat-Glasummantelung ist.

7. Eingebetteter Sensor (1) zur kontinuierlichen Messung einer mechanischen Festigkeit von Strukturen (6), die aus Zementmaterial gefertigt sind, nach Anspruch 6, **dadurch gekennzeichnet, dass** die Borsilikat-Glasummantelung eine Zusammensetzung umfasst, die ausgewählt ist aus

- 74,5 % $SiO_2$, 15 % $B_2O_3$, 3 % $Na_2O$, 2 % $Al_2O_3$, 1,5 % $K_2O$; und
- 73 % $SiO_2$, 16,5 % $B_2O_3$, 6 % $PbO$, 3 % $Na_2O$, 1,5 % $K_2O$.

8. Eingebetteter Sensor (1) zur kontinuierlichen Messung einer mechanischen Festigkeit von Strukturen (6), die aus Zementmaterial gefertigt sind, nach Anspruch 6, **dadurch gekennzeichnet, dass** die Borsilikat-Glasummantelung eine Zusammensetzung von mindestens 20 bis 25 % Zr umfasst.

9. Eingebetteter Sensor (1) zur kontinuierlichen Messung einer mechanischen Festigkeit von Strukturen (6), die aus Zementmaterial gefertigt sind, nach Anspruch 5, **dadurch gekennzeichnet, dass** der ferromagnetische Kern (3) eine chemische Zusammensetzung umfasst, die ausgewählt ist aus CoFeSiB, FeSiB, FeNiSiB und FeCoSiB mit einem Anteil von Si+B von mehr als 14 % und weniger als 35 % und von Co, Ni und Fe von mehr als 40 %.

10. Eingebetteter Sensor (1) zur kontinuierlichen Messung einer mechanischen Festigkeit von Strukturen (6), die aus Zementmaterial gefertigt sind, nach Anspruch 5, **dadurch gekennzeichnet, dass** der ferromagnetische Kern (3) eine chemische Zusammensetzung umfasst, die ausgewählt ist aus CoSiB und CoFeSiB mit einem Anteil von Si+B von mehr als 15 % und weniger als 35 %, von Co von mehr als 40 % und Fe von weniger als 6 %.

11. System zur kontinuierlichen Messung einer mechanischen Festigkeit von Strukturen (6), die aus Zementmaterial gefertigt sind, basierend auf einem elektromagnetischen Induktionseffekt, **dadurch gekennzeichnet, dass** es umfasst:

- den Sensor (1) nach einem der Ansprüche 1 bis 10, wobei der magnetische Mikrodraht (2) eine hohe und positive Magnetostriktion in einem Bereich von $3e^{-5}$ bis $4e^{-5}$ und eine magnetische Bistabilität umfasst, und wobei der Sensor (1) konfiguriert ist, in Strukturen (6) eingebettet zu werden, die aus Zementmaterial gefertigt sind;
- eine Antriebsspule (7);
- eine innerhalb und konzentrisch zu der Antriebsspule (7) angeordnete Sensorspule (8), die in ihrem Inneren ein umgekehrtes Feld erzeugt;
- eine mit der Antriebsspule (7) verbundene Wechselstromversorgung; und
- ein System zum Lesen und Anzeigen des in der Sensorspule (8) erzeugten umgekehrten Feldes.

12. Verfahren zur kontinuierlichen Messung einer mechanischen Festigkeit in Strukturen (6), die aus Zementmaterial gefertigt sind, basierend auf einem elektromagnetischen Induktionseffekt, der von dem Messsystem nach Anspruch 11 Gebrauch macht, **dadurch gekennzeichnet, dass** es umfasst:

- Erzeugen einer Wechselspannung oder eines Wechselstroms mittels der Wechselstromversorgung, die ein Magnetfeld in der Antriebsspule (7) erzeugt, wobei das Magnetfeld die Ausbreitung einer Magnetwand durch den magnetischen Mikrodraht (2) des Sensors (1) erzeugt, wobei die Magnetwand einen schmalen Spannungsimpuls in einem umgekehrten Magnetfeld der Sensorspule (8) erzeugt,

- Messen des in der Sensorspule (8) erzeugten umgekehrten Magnetfeldes (10) durch ein System zum Lesen und Anzeigen des umgekehrten Magnetfeldes (10),

- Ermitteln der mechanischen Festigkeit der Struktur (6) aus Zementmaterial auf der Grundlage der Messung des umgekehrten Magnetfeldes (10).

13. Verfahren zur kontinuierlichen Messung einer mechanischen Festigkeit in Strukturen (6), die aus Zementmaterial gefertigt sind, basierend auf einem elektromagnetischen Induktionseffekt nach Anspruch 12, **dadurch gekennzeichnet, dass** die Antriebsspule (7) und die Sensorspule (8) außerhalb der Struktur (6) bleiben, die aus Zementmaterial gefertigt ist.

14. Verfahren zur kontinuierlichen Messung einer mechanischen Festigkeit in Strukturen (6), die aus Zementmaterial gefertigt sind, basierend auf einem elektromagnetischen Induktionseffekt nach Anspruch 12, **dadurch gekennzeichnet, dass** die Antriebsspule (7) und die Sensorspule (8) vorher um den Sensor (1) gewickelt und zusammen mit dem Sensor (1) in die Struktur (6) eingebettet werden, die aus Zementmaterial gefertigt ist.

15. Verfahren zur Herstellung des eingebetteten Sensors (1) nach einem der Ansprüche 1 und 3 bis 10, **dadurch gekennzeichnet, dass** es umfasst:

- Herstellen des amorphen magnetischen Mikrodrahts (2) mittels herkömmlicher ultraschneller Abkühl- und Strecktechniken,
- Schützen des magnetischen Mikrodrahts (2) durch eine geeignete Zusammensetzung, so dass er gegen die alkalische Umgebung des Blocks (5) aus Zementmaterial beständig ist,
- Schneiden des magnetischen Mikrodrahts (2) in Abschnitte und Unterziehen desselben einer Wärmebehandlung, die ausgewählt ist aus der Verwendung eines elektrischen Stroms und der Verwendung eines herkömmlichen Ofens, um die innere Struktur des magnetischen Mikrodrahts (2) zu stabilisieren, und Verbessern der Empfindlichkeit des magnetischen Mikrodrahts (2) gegenüber angelegten mechanischen Spannungen,
- Anwenden einer chemischen Behandlung mittels Säuren auf die geschnittenen Enden des magnetischen Mikrodrahts (2), um gleichmäßige und polierte Enden zu erhalten,

- Einbetten mindestens des magnetischen Mikrodrahts (2) in einen Block (5) aus Zementmaterial; und
- Aushärten des Blocks (5) aus Zementmaterial, wobei die Entwicklung seiner mechanischen Eigenschaften mittels zerstörungsfreier Bewertungstechniken kontrolliert wird.

## Revendications

1. Capteur intégré (1) pour la mesure en continu de la résistance mécanique dans des structures (6) en matériau cimentaire, **caractérisé en ce qu'**il comprend :

- un bloc (5) en matériau cimentaire, configuré pour être intégré dans des structures (6) faites en matériau cimentaire, et
- au moins, un microfil magnétique amorphe (2), intégré dans le bloc (5) en matériau cimentaire, le microfil magnétique (2) étant résistant à l'environnement alcalin du bloc (5) en matériau cimentaire et comprenant un noyau métallique (3).

2. Capteur intégré (1) pour la mesure en continu de la résistance mécanique dans des structures (6) en matériau cimentaire, **caractérisé en ce qu'**il comprend :

- un bloc (5) en matériau cimentaire, configuré pour être intégré dans des structures (6) faites en matériau cimentaire, et
- au moins, un microfil magnétique nanocristalline (2), intégré dans le bloc (5) en matériau cimentaire, le microfil magnétique (2) étant résistant à l'environnement alcalin du bloc (5) en matériau cimentaire et comprenant un noyau métallique (3).

3. Capteur intégré (1) pour la mesure en continu de la résistance mécanique dans des structures (6) en matériau cimentaire, selon l'une quelconque des revendications précédentes, **caractérisé en ce que** le noyau métallique (3) est ferromagnétique.

4. Capteur intégré (1) pour la mesure en continu de la résistance mécanique dans des structures (6) en matériau cimentaire, selon la revendication 3, **caractérisé en ce que** le noyau ferromagnétique (3) comprend un matériau choisi parmi le chrome, le zirconium et une combinaison des deux, afin d'être résistant aux agents corrosifs et oxydants du bloc (5) en matériau cimentaire.

5. Capteur intégré (1) pour la mesure en continu de la résistance mécanique dans des structures (6) en matériau cimentaire, selon la revendication 3, **caractérisé en ce que** le microfil (2) comprend en outre une gaine isolante en verre (4), afin d'être résistant à l'environnement du bloc (5) en matériau cimentaire.

6. Capteur intégré (1) pour la mesure en continu de la résistance mécanique dans des structures (6) en matériau cimentaire, selon la revendication 5, **caractérisé en ce que** la gaine isolante en verre (4) est une gaine en verre borosilicate.

7. Capteur intégré (1) pour la mesure en continu de la résistance mécanique dans des structures (6) en matériau cimentaire, selon la revendication 6, **caractérisé en ce que** la gaine en verre borosilicate comprend une composition choisie parmi

    - 74,5 % $SiO_2$, 15 % $B_2O_3$, 3 % $Na_2O$, 2 % $Al_2O_3$, 1,5 % $K_2O$; et
    - 73 % $SiO_2$, 16,5 % $B_2O_3$, 6 % $PbO$, 3 % $Na_2O$, 1,5 % $K_2O$.

8. Capteur intégré (1) pour la mesure en continu de la résistance mécanique dans des structures (6) en matériau cimentaire, selon la revendication 6, **caractérisé en ce que** la gaine en verre borosilicate comprend une composition d'au moins 20-25 % Zr.

9. Capteur intégré (1) pour la mesure en continu de la résistance mécanique dans des structures (6) en matériau cimentaire, selon la revendication 5, **caractérisé en ce que** le noyau ferromagnétique (3) comprend une composition chimique choisie parmi CoFeSiB, FeSiB, FeNiSiB et FeCoSiB avec une proportion de Si+B supérieure à 14 % et inférieure à 35 %, et de Co, Ni et Fe supérieure à 40 %.

10. Capteur intégré (1) pour la mesure en continu de la résistance mécanique dans des structures (6) en matériau cimentaire, selon la revendication 5, **caractérisé en ce que** le noyau ferromagnétique (3) comprend une composition chimique choisie parmi CoSiB et CoFeSiB avec une proportion de Si+B supérieure à 15 % et inférieure à 35 %, de Co supérieure à 40 % et de Fe inférieure à 6 %.

11. Système pour la mesure en continu de la résistance mécanique dans des structures (6) en matériau cimentaire, sur la base d'un effet d'induction électromagnétique, **caractérisé en ce qu'**il comprend :

    - le capteur (1), selon l'une quelconque des revendications 1 à 10, dans lequel le microfil magnétique (2) comprend une magnétostriction élevée et positive dans une plage de $3e^{-5}$ à $4e^{-5}$, et une bistabilité magnétique, et dans lequel le capteur (1) est configuré pour être intégré dans des structures (6) en matériau cimentaire ;
    - une bobine d'entraînement (7) ;
    - une bobine de détection (8) située à l'intérieur et concentriquement à la bobine d'entraînement (7) qui génère un champ inversé à l'intérieur de celle-ci ;
    - une alimentation en courant alternatif connectée à la bobine d'entraînement (7) ; et
    - un système de lecture et d'affichage du champ inversé généré dans la bobine de détection (8).

12. Procédé pour la mesure en continu de la résistance mécanique dans des structures (6) en matériau cimentaire sur la base d'un effet d'induction électromagnétique en utilisant le système de mesure défini dans la revendication 11, **caractérisé en ce qu'**il comprend :

    - générer une tension alternative ou un courant alternatif au moyen de l'alimentation en courant alternatif qui génère un champ magnétique dans la bobine d'entraînement (7) où ledit champ magnétique génère la propagation d'une paroi magnétique à travers le microfil magnétique (2) du capteur (1), dans lequel ladite paroi magnétique génère une impulsion de tension étroite dans un champ magnétique inversé de la bobine de détection (8),
    - mesurer le champ magnétique inversé (10) généré dans la bobine de détection (8) par l'intermédiaire d'un système de lecture et d'affichage du champ magnétique inversé (10),
    - établir la résistance mécanique de la structure (6) en matériau cimentaire sur la base de la mesure du champ magnétique inversé (10).

13. Procédé pour la mesure en continu de la résistance mécanique dans des structures (6) en matériau cimentaire sur la base de l'effet d'induction électromagnétique, selon la revendication 12, **caractérisé en ce que** la bobine d'entraînement (7) et la bobine de détection (8) restent à l'extérieur de la structure (6) en matériau cimentaire.

14. Procédé pour la mesure en continu de la résistance mécanique dans des structures (6) en matériau cimentaire sur la base de l'effet d'induction électromagnétique, selon la revendication 12, **caractérisé en ce que** la bobine d'entrainement (7) et la bobine de détection (8) sont enroulées au préalable autour du capteur (1) et intégrées, conjointement avec le capteur (1), dans la structure (6) en matériau cimentaire.

15. Procédé de fabrication du capteur intégré (1) défini dans l'une quelconque des revendications 1 et 3 à 10, **caractérisé en ce qu'**il comprend :

    - produire le microfil magnétique amorphe (2) au moyen de techniques classiques de refroidissement ultra-rapide et d'étirement,

- protéger le microfil magnétique (2) au moyen d'une composition appropriée, de sorte qu'il résiste l'environnement alcalin du bloc (5) en matériau cimentaire,

- découper le microfil magnétique (2) en sections et le soumettre à un traitement thermique choisi entre l'utilisation d'un courant électrique et l'utilisation d'un four classique pour stabiliser la structure interne dudit microfil magnétique (2), et améliorer la sensibilité du microfil magnétique (2) aux contraintes mécaniques appliquées,

- appliquer un traitement chimique au moyen d'acides aux extrémités coupées du microfil magnétique (2), afin d'obtenir des extrémités uniformes et polies,

- intégrer au moins le microfil magnétique (2) dans un bloc (5) en matériau cimentaire ; et

- durcir le bloc (5) en matériau cimentaire en contrôlant l'évolution de ses propriétés mécaniques au moyen de techniques d'évaluation non destructives.

FIG.1

FIG.2

FIG.2a

**FIG.3**

FIG.4a

FIG.4b

FIG.5a

FIG.5b

**FIG.6a**

FIG.6b

FIG.6c

FIG.6d

FIG.7a

FIG.7b

FIG.8a

FIG.8b

FIG.8c

FIG.8d

FIG.8e

22

**FIG.9**

23

M

H

**FIG.9a**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012230365 A1
- WO 2007116218 A1
- US 2007144618 A1
- US 7771545 B2

**Non-patent literature cited in the description**

- **V ZHUKOVA.** Magnetic and Mechanical Properties of Magnetic Glass-Coated Microwires with Glass Coating. *Materials Science Forum,* vol. 480 (481), 293-298, www.scientific.net/MSF.480-481.293